# EUROPEAN PATENT APPLICATION

(11) **EP 1 698 591 A1**
(43) Date of publication of application: **06.09.2006**
(21) Application number: 05380022.3
(22) Date of filing: 08.02.2005
(51) Int. Cl.: C01B 13/10, C01B 13/11, A61L 2/18, A61L 2/20

(54) **Apparatus for producing ozonized water**

(71) Applicant: Zasatec, S.L., 49032 Zamora (ES)
(72) Inventor: Escudero Sánchez, Francisco, 37004 Salamanca (ES)
(74) Representative: Vicario Cubillo, Marcos

(57) **Abstract**

The prophylaxis method is based on the application of ozonized water for cleaning and disinfection treatments, for which a device is used embodied in a water tank (9) at which the required ozone arrives through a diffuser (10), said gas being produced by the ozone generator (2) fed with an air and oxygen flow; said tank (9) is provided with a level probe (16) for controlling the system with the aid of a processor (1) and a pressure gauge (18) that regulate the correct operation of the apparatus: The assembly is provided with an information and control display (21) to show the system state and an apparatus-user interface (22) to allow the user (23) to manage and control the apparatus.

## Description

### OBJECT OF THE INVENTION

The present invention relates to a method for applying ozonized water, specifically conceived for its use in medical, veterinary and similar treatments.

The present invention also provides the apparatus needed to perform said method.

The object of the invention is thus to provide a new prophylaxis method and the technical equipment needed for its automatic application, based on the use of ozonized water that is produced in a continuous manner, which allows obtaining optimum results in the treatments in which it is used.

### BACKGROUND OF THE INVENTION

Prophylaxis methods are known in the fields of medicine, veterinary science and the like, used to carry out hygiene, cleaning or disinfection operations that are so necessary in this area, based on applying products or varying effectiveness.

In this sense, in the field of stomatology the most commonly used application is a bicarbonate solution in water, which among other drawbacks is not easily tolerated by all patients due to its organoleptic characteristics.

The same occurs with products of different types used in other medical or veterinary treatments for this type of cleaning, disinfection or similar applications.

On another hand, the effectiveness of these products is not entirely satisfactory, while its production and application generally causes problems of various types. For these reasons, a new product has been searched for that can be applied automatically, facilitating its use in the various treatments and that allows obtaining optimum results.

### DESCRIPTION OF THE INVENTION

The method for applying ozonized water in medical, veterinary and similar treatments disclosed by the invention, together with the apparatus therefor, which is also an object of the invention, solves the aforementioned drawbacks in a fully satisfactory manner in all of the aspects discussed.

In a general sense, the method consists of the application of ozonized water for conducting various prophylaxis treatments, using an apparatus that allows producing said water in a continuous manner; as well as providing means that allow an efficient control of the ozonized water outlet and generation.

The apparatus for performing the prophylaxis method for medical, veterinary and similar treatments proposed by the invention consists of three clearly differentiated systems: an electronic system, a hydraulic system and a user-apparatus interface. The assembly allows, in first place, to produce ozonized water that will be taken to the location of application, where it will be outlet by appropriate systems to perform the prophylaxis treatment for which it is intended.

The electronic system shall be in charge of controlling the aspects conceming production and outlet of the ozonized water, as well as establishing the safety and maintenance of the apparatus. Its main element is a processor that communicates with various inputs and outputs that control the apparatus. Said inputs and outputs are associated to various elements or control, safety and information systems that are in tum in communication with the processor, allowing it to know the system state at all times and act accordingly, giving the corresponding instructions in each case to prevent system failures, and if these occur to allow their simple and quick detection, indicating this to the user so that the appropriate actions may be taken.

The hydraulic system shall be in charge of producing ozone first, which will then be mixed with water to obtain the ozonized water that will be taken in suitable conditions to the outlet provided for its application in the medical, veterinary treatment or the like by the specialist in charge of performing said treatment.

For ozone production, the system is provided with air and pure oxygen inputs that are taken to an ozone generator where the ozone required will be produced by, for example, an UV lamp or arc lamp to later produce the ozonized water.

This ozone produced will be taken to a tank previously filled with water; the gas is mixed with the water with a diffuser introduced in the tank. This allows obtaining the ozonized water, which will be impelled by a pump to the corresponding outlet provided for it, i.e. until its application in the aforementioned treatment when the user determines.

Inside the tank provided for mixing the water with the ozone produced, the system is fitted with a level gauge to control the height of liquid in the tank to prevent it from being excessively filled, causing possible spillage due to overflow. The probe is connected to the processor which, in view of the information transmitted by said probe, acts on the valves disposed at the system's water inlet, opening or closing them according to the signal transmitted. One of these valves is a spillage or safety valve that allows shutting the water inlet to the system when any failure is detected in the liquid level in the tank; this is, if the water in the tank overflows said safety valve will close, thereby preventing more water from entering the system.

The system is also provided with a pressure gauge that will also be connected to the central processor that controls the entire apparatus. This pressure gauge is meant to detect the pressure variations occurring in the system so that, in case of a pressure increase at the ozonized water outlet, it sends a signal to the processor to stop pumping the liquid. Similarly, if the pressure at said point drops the pressure gauge will send the corresponding signal to the processor so that it can act on the system to reinitiate the pumping the liquid and thereby the entire ozonized water production process.

Both the water and the air inlets are provided with appropriate filters that prevent access to the system of impurities of various types that may be detrimental to its object, causing obstructions in the ducts or contaminating the ozonized water which due to the nature of its application must be totally purified.

The assembly is completed, as mentioned above, with a user-apparatus interface that allows establishing the communication between the user and the apparatus to perform any required modifications or establish any instructions considered convenient by connection with the central system processor.

As may be appreciated, the systems associated to the operation of the apparatus are govemed and controlled by a common element, the processor. It is therefore an easy system to use, that establishes a number of controls to prevent failures in the components. In any case, if there were any failures in any of the components of the apparatus the system will be prepared to detect and communicate this, displaying an error message in a control screen that is connected to the processor.

### DESCRIPTION OF THE DRAWINGS

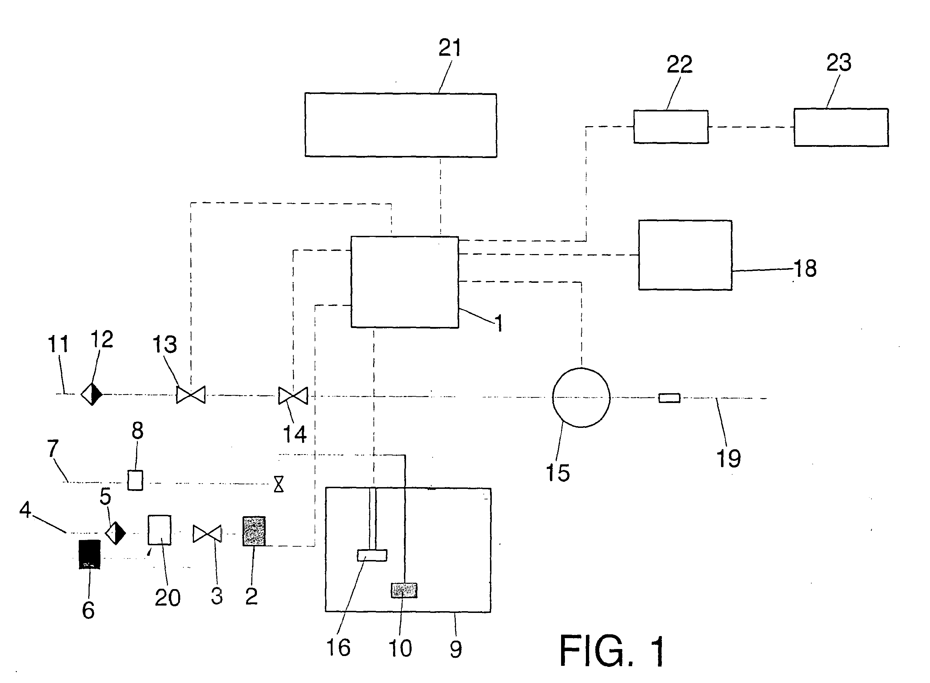

To complete the description being made and in order to aid a better understanding of the characteristics of the invention, according to an example of a preferred embodiment, a single sheet of drawings is accompanied as an integral part of the description where for purposes of illustration and in a non-limiting sense its only figure shows a schematic block diagram of the system for performing the method for applying ozonized water in medical, veterinary and similar treatments proposed by the invention.

### PREFERRED EMBODIMENT OF THE INVENTION

In view of the aforementioned figure, it can be seen that the system for performing the method for applying ozonized water for medical, veterinary and similar treatments proposed by the invention consists of various elements of a hydraulic and electronic nature associated to a processor (1) that controls all system actions by inputs/outputs with associated control, safety and information systems.

The prophylaxis method proposed by the aforementioned treatments consists of the application for operations such as cleaning or disinfection of said ozonized water delivered to the patient, where it is provided with an outlet for the ozonized water produced, as appropriate for each type of application.

The system can provide the ozonized water needed to apply the treatment. To do so, it first produces the ozone that will later be used to ozonize the water. This gas is produced by an ozone generator (2), of the type for example that include a UV lamp or an arc lamp that will be in charge of activating the oxygen that arrives at the generator, transforming it into ozone. A flow or air or pure oxygen arrives at said ozone generator (2) through a valve (3). This air flow (4) first passes through a filter (5) where any impurities present in the air will be retained, and before it enters the ozone generator (6) pure oxygen will be released by a distributor (6).

In the event of a possible excess in the ozone production, this is, if more ozone is produced than needed to apply in the treatment at a given time, the system has an outlet duct (7) for this excess gas, but instead of releasing it directly to the atmosphere, thereby polluting it, it is made to react with a catalyst (8) to convert it into oxygen, which can be released without polluting the environment.

The ozone produced in the generator (2) enters a tank (9) that has been previously filled with water through an ozone diffuser (10) that is introduced in the tank (9). The water arrives at said tank (9) through a duct (11) in which, before the inlet to the tank (9), a filter (12) is fitted meant to clarify the water and remove any impurities contained in it that may contaminate it.

The water inlet to the tank (9) is regulated by two valves: a normal operation valve (13) and a safety or overflow valve (14). The latter valve, as previously explained, is meant to cut off the water supply to the tank (9) in case the liquid level reached in it is too high, possibly even overflowing. The valves (13) and (14) are both connected to the central processor (1), which will be in charge of transmitting the appropriate instructions to allow or prevent water passage through the duct (11) to the tank (9).

The assembly is also provided with a peristaltic pump (15) to start up the hydraulic system, pumping the ozonized water toward its outlet. Said pump (15) is also connected to the processor (1), which is in charge of controlling its operation in view of the information obtained from various points of the system, determining its start up or shut down.

To establish the control of the apparatus, a probe is placed inside the tank (9) connected to the processor (1), to which it sends information on the measured data. Specifically, the probe is a level probe (16) in charge of measuring the level of liquid in the tank (9) and transmitting this information to the processor (1) so that it can act accordingly, sending the appropriate commands to the valve (13) to open allowing liquid to pass until the water level in the tank (9) is correct, closing it when this is achieved, and in case there is a failure in the system and the tank exceeds its safety level to act on the safety valve (14) to cut off the water supply.

To collaborate in the operation control tasks, the system is provided with a pressure gauge (18) located at the ozonized water outlet (19) that is also connected to the processor (1), to which it sends information on the pressure variations that it senses so that, in view of these, the processor (1) can act on the remaining system elements. In this way, if there is a pressure increase at the ozonized water outlet (19), for example because the user is not applying the treatment at this moment, this will be detected by the pressure gauge (18), which will transmit this information to the processor (1), which in tum will give the appropriate instructions to he hydraulic system to make the pump (15) stop pumping liquid, the remaining system elements acting in consequence. If on the contrary the pressure gauge (18) detects a pressure drop, the processor (1) will order the start up of the liquid pump and therefore the rest of the system.

The operation of the apparatus is simple: when the processor (1) is started it sends a signal to the peristaltic pump (15) to fill the water tank (9), turn on the UV or arc lamp of the ozone generator (2) to begin ozone production and activate the compressor (20) to initiate circulation of the gas to the ozone dispenser (10) placed inside the water tank (9). At this time the information and control screen (21) coupled to the apparatus will show the message that the system is ready for operation. The time required until it reaches the optimum state is approximately five minutes.

To allow establishing communication of the apparatus with the user, to establish the control parameters and other functions required for a correct operation of the assembly, a user-apparatus interface (22) is provided that allows the user (23) to control and manage the apparatus.

## Claims

1. Method for applying ozonized water in medical, veterinary and similar treatments, **characterised in that** it is based on the use of ozonized water as a cleaning and disinfectant product.

2. Apparatus for performing the method for applying ozonized water in medical, veterinary and similar treatments, as claimed in claim 1, **characterised in that** it can generate the ozone required to ozonize the water with the aid of an ozone generator (2) using a flow of air and oxygen, said ozone being sent to a tank (9) that has been previously filled with water, which it reaches through an ozone diffuser (10), such that a level probe (16) is fitted in said tank (9) connected to a processor (1) that controls the system, regulating according to the information transmitted to it both the oxygen and water input by means of the valves (3) and (13) respectively, as well as the outlet of ozonized water(19) with the aid of a pressure gauge (18) installed at said outlet and also connected to the processor (1), which acts on a pump (15) and on the remaining elements of the system to allow or prevent the pumping of gas or liquid to the system or to the outlet.

3. Apparatus for performing the method for applying ozonized water in medical, veterinary and similar treatments, as claimed in claim 2, **characterised in that** both the air inlet (4) and the water inlet (11) have appropriate filters (5) and (12) to prevent the entry of contaminants in the system.

4. Apparatus for performing the method for applying ozonized water in medical, veterinary and similar treatments, as claimed in claim 2, **characterised in that** the water duct (11) is provided with at least one safety or overflow valve (14) connected to the processor (1), which is in charge of transmitting the closing order if, in the case of a system failure, the tank reaches a high liquid level.

5. Apparatus for performing the method for applying ozonized water in medical, veterinary and similar treatments, as claimed in claim 2, **characterised in that** the system is provided with an outlet (7) for the excess ozone produced that incorporates a catalyst (8) to transform said ozone into oxygen before it is released to the atmosphere.

6. Apparatus for performing the method for applying ozonized water in medical, veterinary and similar treatments, as claimed in claim 2, **characterised in that** the apparatus is provided with an information and control display (21) connected to the processor (1), to show the user data relating to the system status.

7. Apparatus for performing the method for applying ozonized water in medical, veterinary and similar treatments, as claimed in claim 2, **characterised in that** the assembly is completed by a user-apparatus interface (22) also connected to the processor (1), which allows the user (23) to interact with the system from the outside to manage and control the apparatus.

## Amended claims

### Amended claims in accordance with Rule 86(2) EPC.

**1.** Water ozonation apparatus for use in medical and veterinary treatments, which comprising a tank (9) that contains water obtained from a duct or source (11), an ozone generator (2) which receives pure oxygen through a duct (4), wherein the ozone produced in the generator (2) is applied to the water of the tank (9) to ozonize said water, the tank (9) having an outlet (19) for ozonized water intended as a cleaning or prohylactic product applicable in medical or veterinary treatments and the like, **characterised in that** it also comprises a processor (1) to control the system operation and a filter (5) in the duct (4) that feeds ozone to the generator (2), an outlet (7) combined with a catalyst (8) to let out the excess ozone generated in the generator (2), also including safety valves (13) and (14) as well as a prior filter (12) interposed in the water supply duct (11) to the tank (9); with the special characteristic that at the ozonized water outlet (19) a peristaltic pump is provided for impelling said water, as well as a pressure gauge (18) to stop or activate the pump (15) according to the higher or lower pressure of ozonized water at the outlet (19), also incorporating a level gauge (16) at the tank (9) which, as are the valves (13-14) of the water supply duct (11), the valve (3) provided in the ozone supply duct (4), the peristaltic pump (15) and the pressure gauge (18), is connected to the processor (1) to control the operation of the system.

**2.** Water ozonation apparatus for use in medical and veterinary treatments, according to claim 1, **characterised in that** filters (5) and (12) are included both at the pure oxygen inlet duct (4) and at the water supply duct (11) to the tank (9) in order to prevent entry in the system of contaminants or impurities.

**3.** Water ozonation apparatus for use in medical and veterinary treatments, according to claim 1, **characterised in that** the water supply duct (11) is provided with at least one safety or overflow valve (14) connected to the processor (1), meant to transmit the closing order in the event that a system failure causes a high liquid level in the tank (9).

**4.** Water ozonation apparatus for use in medical and veterinary treatments, according to claim 1, **characterised in that** the catalyst (8) provided at the outlet (7) for the excess ozone produced in the generator (2) transforms said ozone into oxygen before it is released to the atmosphere.

**5.** Water ozonation apparatus for use in medical and veterinary treatments, according to claim 2, **characterised in that** a transformation and control display (21) is included connected to the processor (1) that shows the user data related to the system status.

**6.** Water ozonation apparatus for use in medical and veterinary treatments, according to claim 2, **characterised in that** the assembly is completed by a user-device interface (22) connected to the processor (1) that provides an external connection allowing the user (23) to manage and control the device .

**7.** Water ozonation method, **characterised in that** it comprises the following phases:
- Generating the ozone required to ozonize the water;
- Supplying the ozone to the water contained in a vessel to ozonize said water;
- Passing the pure oxygen from which the ozone is obtained through a filter, a compressor and a valve, discharging impurities retained by the filter to the outside through a distributor;
- Introducing the ozone in the water contained in the vessel through a diffuser;
- Prior feeding of the water in the vessel after passing through a filter and safety valves;
- Impelling the ozonized water towards an outlet towards- a peristaltic pump;
- Controlling the pressure at the ozonized water outlet; and
- Controlling the system operation by a processor that generates the appropriate orders upon receiving the corresponding signals from the component parts of said system.
